Europäisches Patentamt

European Patent Office

Office européen des brevets

(19)

(11) Publication number: 0 146 929
A2

## (12) EUROPEAN PATENT APPLICATION

(21) Application number: 84115897.5

(22) Date of filing: 20.12.84

(51) Int. Cl.⁴: C 12 P 13/24
C 12 N 15/00, C 12 N 1/20
//(C12P13/24, C12R1:43),
(C12N1/20, C12R1:43)

(30) Priority: 28.12.83 JP 247867/83

(43) Date of publication of application:
03.07.85 Bulletin 85/27

(84) Designated Contracting States:
CH DE FR GB LI

(71) Applicant: Tanabe Seiyaku Co., Ltd.
No. 21 Dosho-machi 3-chome Higashi-ku
Osaka-shi Osaka-fu(JP)

(72) Inventor: Kisumi, Masahiko
No. 12-20, Morikita-cho 4-chome Higashinada-ku
Kobe-shi Hyogo-ken(JP)

(72) Inventor: Sugiura, Masaki
No. 3-19 Seiwadai-Higasi 5-chome
Kawanishi-shi Hyogo-ken(JP)

(72) Inventor: Takagi, Tsutomu
No. 6-33-609 Nishi-Midorigaoka 1-chome
Toyonaka-shi Osaka-fu(JP)

(72) Inventor: Imai, Yuji
N. 13-3-204, Sanjyocho
Ashiya-shi Hyogo-ken(JP)

(74) Representative: Hansen, Bernd, Dr.rer.nat. et al,
Hoffmann, Eitle & Partner Patentanwälte Arabellastrasse 4
D-8000 München 81(DE)

(54) Microorganism of the genus Serratia containing a hybrid plasmid and process for preparing the same.

(57) A novel microorganism of the genus *Serratia* containing a hybrid plasmid prepared by joining a vector plasmid to a chromosomal fragment encoding enzymes of proline synthesis which is obtained from a chromosomal DNA of a microorganism of the genus *Serratia* having L-proline-producing activity. A method for producing L-proline by using said novel microorganism is also disclosed.

EP 0 146 929 A2

Microorganism of the genus Serratia containing a hybrid
plasmid and process for preparing the same

This invention relates to a novel microorganism and a
method for producing L-proline.  More particularly, it
relates to a novel microorganism of the genus Serratia and a
method for producing L-proline by using said microorganism.

L-proline is an important amino acid useful as a drug
or food additive  and is prepared by using various microorganisms of the genera Brevibacterium, Micrococcus, Kruthia,
Saccharomyces, Bacillus, Escherichia and so forth.  However,
wild strains of microorganism of the genus Serratia do not
produce and accumulate L-proline because said strains have
potent L-proline-degrading enzyme activity and, at the same
time,are subject to metabolic regulation in L-proline biosynthesis.

We have previously found that mutants derived from a
microorganism of the genus Serratia, for example, a mutant
which is defective in a L-proline-degrading enzyme and
resistant to a proline analog and/or purine analog and a
mutant which is defective in a L-proline-degrading enzyme and resistant to a proline analog under increased osmotic pressure, have L-proline-producing activity (Japanese Patent Publication (unexamined) Nos. 60995/1983 and 224696/1983).

As a result of various investigations, we have now found that a microorganism of the genus Serratia having more potent L-proline-producing activity than that of the above-mentioned known mutant of the genus Serratia can be obtained by isolating a chromosomal DNA (deoxyribonucleic acid) fragment encoding enzymes of proline synthesis from said known mutant, joining a vector plasmid to said chromosomal DNA fragment and then introducing the resultant hybrid plasmid into a microorganism of the genus Serratia.

An object of the present invention is to provide a novel microorganism useful for the industrial production of L-proline. Another object of the present invention is to provide a method for the production of L-proline. These objects as well as other objects will be apparent to these skilled in the art from the following description.

According to the present invention, there is provided a novel microorganism of the genus Serratia containing a hybrid plasmid prepared by joining a vector plasmid to a chromosomal DNA fragment encoding enzymes of proline synthesis which is obtained from a chromosomal DNA of a microorganism of the genus Serratia having L-prolin-producing activity. There is also provided a method for producing L-proline which comprises cultivating microbial cells of the above-mentioned novel microorganism in a nutrient medium to produce and accumulate L-proline in the medium and then recovering the L-proline therefrom.

Throughout the specification and claims, the chromosomal DNA fragment encoding enzymes of proline synthesis stands for deoxyribonucleic acid (DNA) carring at least one of genes for enzymes involved in the synthesis of proline from glutamic acid, i.e., gene for $\gamma$-glutamyl kinase (hereinafter referred to as "proB"), gene for $\gamma$-glutamyl phosphate reductase (hereinafter referred to as "proA") and gene for pyrroline-5-carboxylate reductase (hereinafter referred to as "proC"). Preferable examples of the chromosomal DNA fragment are DNA containing proB, proA and proC and DNA containing proB and proA. It is preferred that proB contained in the chromosomal DNA fragment is a mutation-type gene which is not subject to feedback control.

Preparation of microorganisms of the present invention:
(Chromosomal DNA and preparation thereof)

Microorganisms to be used as a source of the above-mentioned chromosomal DNA are not limited to a specific one so far as it is a microorganism of the genus Serratia having L-proline producing activity. Examples of such microorgansms include Serratia marcescens which is defective in a L-proline-degrading enzyme and resistant to a proline analog and/or a purine analog, Serratia marcesecens which is defective in a L-proline-degrading enzyme and resistant to a proline analog under increased osmotic pressure, and the like.  Representative examples of such microorganisms are <u>Serratia</u> <u>marcescens</u> DTr-12 (FERM BP-171) (i.e., a mutant which is defective in L-proline oxidase and resistant to 3,4-dehydro-DL-proline and L-thiazolidine-4-carboxylic acid), <u>Serratia</u> <u>marcescens</u> OAr-80 (FERM BP-173) (i.e., a mutant which is defective in L-proline oxidase and resistant to L-azetidine-2-carboxylic acid in the presence of a high concentration of sodium chloride), and the like.

Isolation of chromosomal DNA from the above-mentioned microorganism may be accomplished by a conventional method, for example, by lysing microbial cells of said microorganism with lysozyme and a detergent [e.g., SDS (chemical name: sodium dodecyl sulfate), sarcosil (chemical name: sodium N-lauroylsarcosinate), etc.], deproteinizing the lysate and then precipitating chromosomal DNA with ethanol (J. Mol. Biol., <u>3</u>, 208(1961); Biochem. Biophys. Acta, <u>72</u>, 619(1963)). (Vector plasmid DNA and preparation thereof)

The vector plasmid to be joined to the chromosomal DNA fragment is not limited to a specific one so far as it is capable of being introduced into a microorganism of the genus <u>Serratia</u> and is replicable in said microorganism. Examples of such vector plasmid include pACYC177 (J. Bacteriol., <u>134</u>, 1141(1978)), pBR322 (Gene, <u>2</u>, 95(1977)), pBR313 (Gene, <u>2</u>, 75(1977)), pBR325 (Gene, <u>4</u>, 121(1978)), pMB1 (Fed. Proc., <u>35</u>, 2973(1976)), mini F-derived plasmids

such as pKP1155 (Mol. Gen. Genet., $\underline{191}$, 231 - 237 (1983)) [plasmid pKP1155 being prepared by joining ampicillin-, chloramphenicol-, spectinomycin- and streptomycin-resistant genes to a mini F (i.e., the fragment containing regions B, C and $A_2$ for replication initiation) obtained from F⁻ plasmid $\underline{Eco}$RI f5 fragment (Proc. Nat. Aca. Sci., $\underline{73}$, 64(1976)], and the like. These plasmids are preferably used for preparation of hybrid plasmid after introducing into a microorganism of the genus $\underline{Serratia}$ and then extracting from said microorganism by a conventional method.

(Preparation of hybrid plasmid)

The hybrid plasmid may be prepared from the chromosomal DNA and the vector plasmid by a conventional method [e.g., Method in Enzymology, $\underline{68}$, 41(1979); "IDENSHI SOSA JIKKEN HO" (Experimental Method in Genetic Manipulation), Ed. by Y. Takagi, Kodan-sha Scientific Co., Japan, 135 - 159(1980)]. For example, the hybrid plasmid is prepared by treating the chromosomal DNA and the vector plasmid DNA with a restriction endonuclease (e.g., $\underline{Hind}$III, $\underline{Pst}$I, $\underline{Bam}$HI, etc.) to cut the DNA strands and then treating the resultant DNA fragments (i.e., chromosomal DNA fragment and vector plasmid DNA fragment) with a ligase (e.g., $T_4$ DNA ligase, $\underline{Escherichia}$ $\underline{coli}$ ligase, etc) to join the DNA strands. Depending upon the nature of the termini of the DNA fragments, the hybrid plasmid may be prepared by treating the DNA fragments with terminal transferase, DNA polymerase, etc. and then treating with a ligase to join the DNA strands.

- 6 -                                        0146929

The hybrid plasmid thus obtained may be used for transformation as it is.   In order to facilitate the selection of a transformant containing the desired hybrid plasmid, however, the hybrid plasmid obtained above is preferably used after introducing the hybrid plasmid obtained above into a mutant, selecting microbial cells containing the desired hybrid plasmid and then extracting said plasmid therefrom by a conventional method.   The mutant to be used for this purpose includes, for example, microorganisms of the genus Serratia or Escherichia which do not show proline-producing activity or are sensitive to antibiotics (e.g., ampicillin, kanamicin).

(Host microorganisms)

The host microorganism into which the hybrid plasmid is introduced is not limited to a specific one so far as it is a transformable microorganism of the genus Serratia.   For example, the microorganisms used as the source of the chromosomal DNA are preferably used as the host microorganism. Wild strains of Seratia marcescens are also used as the host microorganism.

(Transformation by hybrid plasmid)

The transformation of the host microorganism by the hybrid plasmid may be carried out by a conventional method, for example, by treating microbial cells of the host microorganism with a calcium chloride solution to increase the permeability of the cell wall, and then introducing the hybrid plasmid DNA into said microorganism (J. Mol. Biol., 53, 159(1970)).    Proline-producing microbial cells are selected among the colonies of the transformant obtained above.    Thus, there is obtained the microorganism of the present invention, i.e., the microorganism of the genus Serratia containing a hybrid plasmid prepared by joining a vector plasmid to a chromosomal DNA fragment carrying the gene for L-proline synthesis which is obtained from a chromosomal DNA of a microorganism of the genus Serratia having L-proline-producing activity.

The representative microorganisms of the present invention have been deposited with Fermentation Research Institute (FRI), Agency of Industrial Science and Technology, Ibaraki-ken, Japan under Budapest Treaty on 12th December, 1983. They are Serratia marcescens TA5006 (FERM BP-649), Serratia marcescens TA5007 (FERM BP-650), Serratia marcescens TA5008 (FERM BP-651) and Serratia marcescens TA5009 (FERM BP-652). (Productin of L-proline)

Since the microorganism of the present invention has a potent L-proline-producting activity, L-proline can be prepared by using said microorganism.

That is, L-proline can be prepared by cultivating the microorganism of the present invention in a nutrient medium to produce and accumulate L-proline in a nutrient medium and then recovering the L-proline therefrom.

As the medium to be used for production of L-proline, there may be used a conventional nutrient medium containing 10 to 20 w/v % of carbon sources such as saccharides (e.g., glucose, sucrose, molasses), organic acids (e.g., succinic acid, citric acid, fumaric acid) or sugar alcohols (e.g., glycerol); 1 to 5 w/v % of nitrogen sources such as organic ammonium salts (e.g., ammonium fumarate, ammonium succinate), inorganic ammonium salts (e.g., ammonium sulfate, ammonium chloride) or urea; 0 to 1 w/v % of organic nutrients such as corn steep liquor, peptone or yeast extract; and a small amount of inorganic substances such as potassium phosphate, magnesium sulfate or ferrous sulfate. In order to adjust the medium to pH 6 to 8, calcium carbonate, ammonia, citric acid and the like may be added to the medium. Further, a precursor for L-proline biosynthesis (e.g., L-glutamic acid, L-aspartic acid) may be added to the medium.

The cultivation of the microorganism of the present invention may be carried out by a conventional method, for example, by inoculating microbial cells of said microorganism in the nutrient medium and incubating at 25° to 40°C for 2 to 6 days under aerobic conditions.

L-proline thus produced and accumulated in the medium may be recovered by a conventional method, for example, by removing microbial cells and other insoluble materials from the culture medium, passing the resultant solution through a column packed with an ion-exchange resin to adsorb L-proline eluting the adsorbed L-proline with an aqueous ammonia solution, condensing the eluate and then precipitating crystals of L-proline therefrom.

The following Examples further illustrate the present invention in detail but are not to be construed to limit the scope thereof. In Examples, the production of L-proline was confirmed by paper chromatography using ninhydrin reaction and isatin reaction and the quantitative analysis thereof was carried out by a bioassay using Leuconostoc mesenteroides P-60.

Example 1

(1) Preparation of chromosomal DNA:

Serratia marcescens OAr-80 (FERM BP-173) was inoculated into L-broth (one liter; peptone 1 %, yeast extract 0.5 %, sodium chloride 0.5 %; pH 7.2) and incubated with shaking at 30°C for 4 hours. After incubation, microbial cells in the logarithmic growth phase were collected by centrifugation. The microbial cells were lysed by lysozyme treatment and SDS treatment. The lysate was deproteinized by phenol treatment and then treated with ethanol to precipitate chromosomal DNA. The precipitated chromosomal DNA was purified by treating with RNase (final concentration: 10 μg/ml) at 37°C for one hour to obtain the desired chromosomal DNA (8.6 mg).

(2) <u>Preparation of vector plasmid DNA</u>:

<u>Serratia</u> <u>marcescens</u> Sr41 (FERM BP-487) was subjected to mutagenesis in accordance with the method described in Molec. Gen. Genet., <u>124</u>, 197(1973) to give an extracellular nuclease-defective and restriction endonuclease-defective mutant. Then, the mutant was subjected to mutagenesis with N-methyl-N'-nitro-N-nitrosoguanidine, spread on L-broth agar medium containing 5 µg/ml of ampicillin and then incubated at 30°C for one day. Among the resultant colonies (about 100 colonies per plate), a strain which showed higher ampicillin-sensitivity than that of the parent strain was selected to obtain an extracellular nuclease-defective, restriction endonuclease-defective and ampicillin-sensitive mutant. The mutant thus obtained was inoculated into L-broth (50 ml) and incubated with shaking at 30°C. The microbial cells grown exponentially were collected and suspended in an ice-cold 0.1 M calcium chloride-0.5 M sucrose solution (25 ml). The suspension was allowed to stand at 0°C for 30 minutes. The microbial cells were collected and suspended again in an ice-cold 0.1 M calcium chloride-0.5 M sucrose solution (5 ml). Plasmid pKP 1155 (1 µg) obtained from <u>Escherichia</u> <u>coli</u> K-12 was added to the cell suspension (0.2 ml), and the mixture was allowed to stand at 0°C for one hour. After the mixture was heated at 42°C for 2 minutes, L-broth (2 ml) was added thereto. The mixture was incubated at 30°C for 90 minutes. The resultant culture broth (0.4 ml) was inoculated into L-broth agar medium containing 50 µg/ml of ampicillin and incubated at. 30°C for one day. The resultant colonies were isolated to obtain Serratia marcescens containing pKP1155.

The microbial cells thus obtained were inoculated into L-broth (4 liters) and incubated at 30°C for 18 hours. Then, the microbial cells were collected by centrifugation and lysed by lyzozyme treatment and SDS treatment. Sodium chloride was added to the lyzate at a final concentration of 1 M, and the mixture was centrifuged. The supernatant solution was subjected to phenol-treatment, and ethanol was added thereto. The mixture was centrifuged to precipitate DNA. The precipitated DNA was dissolved in a 10 mM Tris HCl-1mM EDTA solution (pH 7.5), and the solution was subjected to cesium chloride-ethidium bromide equilibrium density gradient centrifugation to separate and purify the plasmid DNA. Thus, pKP1155 plasmid DNA (0.2 mg) was obtained.

(3) Preparaton of hybrid plasmid:

Each of the chromosomal DNA (6 µg) obtained in paragraph (1) and the plasmid DNA (1 µg) obtained in paragraph (2) was treated with restriction endonuclease BamHI under normal conditions to cut the DNA strands completely. After heat treatment at 65°C for 10 minutes, both reaction mixtures were combined and treated with $T_4$ DNA ligase under normal conditions to join the DNA strands.

- 12 -

0146929

(4) <u>Selection of hybrid plasmid</u>:

(a)   <u>Escherichia</u> <u>coli</u> HB101 (ATCC 33694) (a Ɣ-glutamyl phosphate reductase-defective and restriction endonuclease-defective strain) was inoculated into L-broth (50 ml) and incubated with shaking at 37°C until the growth thereof reached the middle of the logarithmic growth phase. The microbial cells were collected, washed with an ice-cold 0.1 M magnesium chloride solution (50 ml) and suspended in a 0.1 M calcium chloride solution (25 ml).   The suspension was allowed to stand at 0°C for 30 minutes.   The microbial cells were collected and suspended in a 0.1 M calcium chloride solution (5 ml).   The hybrid plasmid DNA solution obtained in paragrapn (3) was added to the cell suspension, and said suspension was ice-cooled for 30 minutes and subjected to heat treatment at 42°C for 2 minutes to introduce DNA into the cells.   Then, L-broth (50 ml) was added to the cell suspension, and the mixture was incubated with shaking at 37°C for one hour.   The microbial cells were collected and suspended in a physiological saline solution (5 ml).   A small amount of the cell suspension was inoculated into a medium (glucose 0.5 %, dipotassium hydrogen phosphate 0.7 %, potassium dihydrogen phosphate 0.3 %, ammonium sulfate 0.1 %, magnesium sulfate 7 hydrate 0.01 %, sodium L-glutamate 0.01 M, L-leucine 0.001 M, L-methionine 0.001 M, L-lysine 0.001 M, thiamine 0.0005 %, ampicillin 20 µg/ml, agar 1.5 %) and incubated at 37°C for 3 to 4 days.   The resultant colonies were isolated to obtain a proline-producing strain. The strain was treated in the same manner as described in paragraph (2) to isolate hybrid plasmid DNA.

(b)    The DNA obtained in paragraph (a) was introduced into the extracellular nuclease-defective, restriction endonuclease-defective and ampicillin-sensitive mutant of Serratia marcescens (said mutant being obtained in the same manner as described in paragraph (2)) in the same manner as described in paragrapn (4)-(a). The resultant transformant was spread on L-broth (agar 1.5 %) containing 50 µg/ml of ampicillin, and incubated at 30°C for 24 hours. The resultant colonies were isolated to select microbial cells having proline-producing activity. The microbial cells thus obtained were treated in the same manner as described in paragraph (4)-(a) to isolate hybrid plasmid DNA (pTA506).

(5) Preparation of transformant:

The hybrid plasmid DNA (pTA506) obtained above was introduced into Serratia marcescens DTr-12 (FERM BP-171) in the same manner as described in paragraph (4)-(a). The transformant thus obtained was incubated in L-broth agar medium containing 500 µg/ml of ampicillin to obtain the desired transformant, i.e., Serratia marcescens TA5006 (FERM BP-649).

Alternatively, the hybrid plasmid DNA (pTA506) and Serratia marcescens OAr-80 (FERM BP-173) were treated in the same manner as described above to obtain the desired transformant, i.e., Serratia marcescens TA5007 (FERM BP-650).

In order to confirm that the hybrid plasmid pTA506 contained in the transformants TA5006 and TA5007 has proB and proA, the hybrid plasmid DNA was extracted and isolated from each of said transformants and then introduced into Escherichia coli HB101 in the same manner as described in pragraph (4)-(a). The resultant transformants showed proline-producing activity and, therefore, it was confirmed that the hybrid plasmid contained in each of the transfomants TA5006 and TA5007 has proB and proA.

Example 2

(1) Preparation of hybrid plasmid:

(a) Each of the chromosomal DNA(6 µg) obtained in the same manner described in Example 1-(1) and the vector plasmid pACYC177 DNA (1 µg) obtained in the same manner as described in Example 1-(2) was treated with restriction endonuclease PstI under normal conditions to cut the DNA strands completely. After heat treatment at 65°C for 10 minutes, both reaction mixtures were combined and treated with $T_4$ DNA ligase under normal conditions to join the DNA strands.

(b) Escherichia coliC 600 (ATCC 33525) was subjected to mutagenesis with N-methyl-N'-nitro-N-nitrosoguanidine to obtain proline-requiring mutants. A mutant having no pyrroline-5-carboxylate reductase activity was selected among the proline-requiring mutants.

(c)    The hybrid plasmid DNA obtained in paragraph (a) was introduced into the pyrroline-5-carboxylate reductase-defective mutant obtained in paragraph (b) by calcium chloride treatment in the same manner as described in Example 1-(4). The transformant thus obtained was incubated in a medium (glucose 0.5 %, dipotassium hydrogen phosphate 0.7 %, potassium dihydrogen phosphate 0.3 %, ammonium sulfate 0.1 %, magnesium sulfate 7 hydrate 0.01 %, L-threonine 0.001 M, thiamine 0.0005 %, agar 1.5 %) to select a strain which was able to grow in the medium, and hybrid plasmid was extracted and isolated from said strain in the same manner as described in Example 1-(2).

(d)    The hybrid plasmid DNA obtained in paragraph (c) was introduced into the extracellular nuclease-defective, restriction endonuclease-defective and ampicillin-sensitive mutant of Serratia marcescens (said mutant being prepared in the same manner as described in Example 1-(2)), and an ampicillin-sensitive transformant was isolated.  The transformant was treated in the same manner as described in Example 1-(2) to isolate and purify hybrid plasmid DNA (pTA 507).

(e)    Each of the hybrid plasmid pTA507 (1 µg) obtained in paragraph (d) and the hybrid plamid pTA (1 µg) obtained in Example 1-(4)-(b) was treated with restriction endonucleases HindIII and EcoRI under normal conditions to cut the DNA strands completely.  After heat treatment at 65°C for 10 minutes, both reaction mixtures were combined and treated with T$_4$ DNA ligase under normal conditions to join the DNA strands.

(2) Selection of hybrid plasmid:

(a)   The DNA solution obtained in paragraph (1)-(e) was introduced into the pyrroline-5-carboxylate reductase-defective mutant of Escherichia coli (said mutant being prepared in the same manner as described in paragraph (1)-(b)). The transformant thus obtained was incubated in a medium prepared by adding 20 µg/ml of anpicillin to the medium described in paragraph (1)-(c).   Among the colonies, a proline-producing strain was selected and then treated in the same manner as described in Example 1-(2) to isolate and purify hybrid plasmid DNA.

(b)   The hybrid plasmid DNA obtained in paragraph (a) was introduced into the extracellular endonuclease-defective, restriction endonuclease-defective and ampicillin-sensitive mutant of Serratia marcescens (said mutant being prepared in the same manner as described in Example 1-(2)), and the transformant thus obtained was treated in the same manner as described in Example 1-(2) to isolate and purify hybrid plasmid DNA (pTA 508).

(3) Preparation of transformant:

The hybrid plasmid DNA (pTA508) obtained in paragraph (2)-(b) was introduced into Serratia marcescens DTr-12 (FERM BP-171).   The transformant thus obtained was incubated in L-broth agar medium containing 500 µg/ml of ampicillin to obtain the desired transformant, i.e., _Serratia_ _marcescens_ TA5008 (FERM BP-651).

Alternatively, the hybrid plasmid DNA (pTA508) and _Serratia_ _marcescens_ OAr-80 (FERM BP-173) were treated in the same manner as above to obtain the desired transformant, i.e., _Serratia_ _marcescens_ TA5009 (FERM BP-652).

In order to confirm that the hybrid plasmid DNA (pTA 508) contained in the transformants TA5008 and TA5009 has _proB_, _proA_ and _proC_, the hybrid plasmid DNA was extracted and isolated from each of said transformants and then introduced into each of _Escherichia_ coli which is defective in pyrroline-5-carboxylate reductase and _Escherichia_ _coli_ HB101. The resultant transformants showed proline-producing activity and, therefore, it was confirmed that the hybrid plasmid contained in each of the transformants TA5008 and TA5009 has _proB_, _proA_ and _proC_.

Example 3

Each of _Serratia_ _marcescens_ TA5006 and TA5008 obtained in Examples 1 and 2 was incubated in L-broth medium containing 500 µg/ml of ampicillin at 30°C overnight. A loopful of the microbial cells obtained above was inoculated into a fermentation medium (pH 7.0) (said medium being prepared by dissolving sucrose 18 %, urea 2.5 %, dipotassium hydrogen phosphate 0.1 %, magnesium sulfate 7 hydrate 0.05 %, ferrous sulfate 7 hydrate 0.0002 %, corn steep liquor 0.3

- 18 -                                              0146929

% and calcium carbonate 3 % in 15 ml of distilled water,
charging the solution into a 500 ml-shaking flask and then
sterilizing it at 120°C for 10 minutes.   The glucose added
to the medium was separately sterilized.) and incubated at
30°C for 72 hours under shaking (140 r.p.m., stroke: 7 cm).

Just for comparison, Serratia marcescens DTr-12 (FERM
BP-171) was incubated in a bouillon slant medium at 30°C
overnight, and a loopful of the microbial cells thus obtained
was inoculated into the same fermentation medium as above
and incubated in the same manner as described above.

The amount of L-proline produced in the medium are
shown in the following Table 1.

Table 1

| Microorganisms | Amount of L-proline produced in medium (mg/ml) |
|---|---|
| (The microorganisms of the present invention) | |
| Serratia marcescens TA5006 | 38.3 |
| Serratia marcescens TA5008 | 39.2 |
| (The microorganism used as host) | |
| Serratia marcescens DTr-12 | 25.6 |

Example 4

Each of Serratia marcescens TA5007 and TA5009 was incubated into L-broth medium containing 500 µg/ml of ampicillin at 30°C overnight.   A loopful of the microbial cells obtained above was inoculated into a fermentation medium (pH 7.0) (said fermentation medium being prepared by dissolving sucrose 25 %, urea 2.8 %, dipotassium hydrogen phosphate 0.1 %, magnesium sulfate 7 hydrate 0.05 %, ferrous sulfate 7 hydrate 0.0002 %, corn steep liquor 0.5 % and calcium carbonae 3 % in 15 ml of distilled water, charging the solution into a 500 ml-shaking flask and then sterilizing it at 120°C for 10 minutes.   The sucrose added to the medium was previously sterilized.) and incubated at 30°C for 120 hours under shaking (140 r.p.m., stroke: 7 cm).

Just for comparison, Serratia marcescens OAr-80 (FERM BP-173) was incubated on a bouillon slant medium at 30°C overnight.   A loopful of the microbial cells obtained above was inoculated into the same fermentation medium as described above and incubated in the same·manner as described above.

The amount of L-proline produced in the medium are shown in the following Table 2.

Table 2

| Microorganisms | Amount of L-proline produced in medium (mg/ml) |
|---|---|
| (The microorganisms of the present invention) | |
| Serratia marcescens TA5007 | 81.2 |
| Serratia marcescens TA5009 | 82.3 |
| (The microorganism used as host) | |
| Serratia marcescens OAr-80 | 58.6 |

Example 5

The culture broth (one liter) of Serratia marcescens TA5007 (said broth being obtained in Example 4) was treated by heat and then filtered to remove insoluble materials. The filtrate was passed through a column packed with a cation exchange resin manufactured by Rohm & Haas Company under the tradename "Amberlite IR-120 B $(H^+)$". After the column was washed with water, the adsorbed L-proline was eluted with an aqueous 5 % ammonia solution. The eluate was condensed under reduced pressure, and the condensed solution was cooled. The precipitated crystals were collected by filtration, whereby L-proline (56.8 g) was obtained.

CLAIMS:

1. A microorganism of the genus Serratia containing a hybrid plasmid prepared by joining a vector plasmid to a chromosomal DNA fragment encoding enzymes of proline synthesis which is obtained from a chromosomal DNA of a microorganism of the genus Serratia having L-proline-producing activity.

2. The microorganism of claim 1, which is Serratia marcescens containing the hybrid plasmid.

3. The microorganism of claim 2, wherein the chromosomal DNA fragment is a deoxyribonucleic acid carrying the genes for $\gamma$-glutamyl kinase and $\gamma$-glutamyl phosphate reductase or a deoxyribonucleic acid carrying the genes for $\gamma$-glutamyl kinase, $\gamma$-glutamyl phosphate reductase and pyrroline-5-carboxylate reductase.

4. The microorganism of claim 2, wherein the chromosomal DNA fragment is a deoxyribonucleic acid carrying the genes for $\gamma$-glutamyl kinase and $\gamma$-glutamyl phosphate reductase, and the vector plasmid is pKP1155.

5. The microorganism of claim 2, wherein the chromosomal DNA fragment is a deoxyribonucleic acid carrying the genes for $\gamma$-glutamyl kinase, $\gamma$-glutamyl phosphate reductase

and pyrroline-5-carboxylate reductase, and the vector plasmid is pKP 1155.

6.    The microorganism of claim 2, wherein the _Serratia_ _marcescens_ containing the hybrid plasmid is a member selected from the group consisting of _Serratia_ _marcescens_ TA5006 (FERM BP-649), _Serratia_ _marcescens_ TA5007 (FERM BP-650), _Serratia_ _marcescens_ TA5008 (FERM BP-651) and _Serratia_ _marcescens_ TA5009 (FERM BP-652).

7.    A method for producing L-proline which comprises cultivating a microorganism of the genus _Serratia_ containing a hybrid plasmid prepared by joining a vector plasmid to a chromosomal DNA fragment encoding enzymes of proline synthesis which is obtained from a chromosomal DNA of a microorganism of the genus _Serratia_ having L-proline-producing activity, in a nutrient medium to produce and accumulate L-proline in the medium, and recovering the L-proline therefrom.

8.    The method of claim 7, wherein _Serratia_ _marcescens_ having the hybrid plasmid is cultivated.

9.    The method of claim 8, wherein the _Serratia_ _marcescens_ containng the hybrid plasmid is a member selected from the group consisting of _Serratia_ _marcescens_ TA5006 (FERM BP-649), _Serratia_ _marcescens_ TA5007 (FERM BP-650), Serratia marcescens TA5008 (FERM BP-651) and Serratia marcescens TA5009 (FERM BP-652).

10.    The method of claim 8, wherein the cultivation is carried out at 25 to 40°C for 2 to 6 days under aerobic conditions.

11.    The method of claim 8, wherein the nutrient medium contains 10 to 20 w/v % of a carbon source, 1 to 5 w/v % of a nitrogen source and 0 to 1 w/v % of an organic nutrient, and the pH of said medium is 6 to 8.